(19) Europäisches Patentamt
European Patent Office
Office européen des brevets

(11) Publication number: **0 351 784 B1**

(12) **EUROPEAN PATENT SPECIFICATION**

(45) Date of publication of patent specification: **01.12.93** (51) Int. Cl.$^5$: **A61K 31/70**

(21) Application number: **89113160.9**

(22) Date of filing: **18.07.89**

The file contains technical information submitted after the application was filed and not included in this specification

# (54) Therapeutic use of the isopropyl ester derivative of monosialoganglioside in nervous pathologies with an inflammatory component.

(30) Priority: **19.07.88 IT 4820888**

(43) Date of publication of application:
**24.01.90 Bulletin 90/04**

(45) Publication of the grant of the patent:
**01.12.93 Bulletin 93/48**

(84) Designated Contracting States:
**AT BE CH DE ES FR GB GR IT LI LU NL SE**

(56) References cited:
**EP-A- 0 167 449**

**Arch. Int. Pharmacodyn, vol. 291, Jan.-Febr. 1988, pp. 238-252; M. Amico-Roxas et al.**

**Klin. Mol. Augenheilk., vol. 180, no. 5, 1982, pp. 493-496, F. Enke Verlag, Stuttgart, DE; B. Algan et al.**

**Acta Neuropathol., vol. 62, nos. 1-2, 1983, pp. 45-50, Springer-Verlag 1983; F. Norido et al.**

(73) Proprietor: **FIDIA S.p.A.**
**Via Ponte della Fabbrica 3-A**
**I-35031 Abano Terme (Padova)(IT)**

(72) Inventor: **Della Valle, Francesco**
**Via Cerato 14**
**Padova(IT)**
Inventor: **Romeo, Aurelio**
**Viale Ippocrate, 93**
**I-00161 Roma(IT)**

(74) Representative: **VOSSIUS & PARTNER**
**Postfach 86 07 67**
**D-81634 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

The present invention concerns a new therapeutic application of an ester derivative of $GM_1$ ganglioside and more precisely of the isopropyl ester of $GM_1$.

Methods for preparing ester and amide ganglioside derivatives, pharmaceutical compositions containing the functional derivatives, as well as the therapeutic use of these derivatives in therapies for pathologies involving the peripheral nervous system and the central nervous system are known and described in EP Publication 0 167 449.

The present invention concerns the discovery of other valuable properties of a particular ester of $GM_1$ ganglioside, and more precisely the antiinflammatory and antiexudative properties of the isopropyl ester of $GM_1$ ganglioside.

Gangliosides are a group of glycosphingolipids with a structure containing a saccharide part bound to a ceramide and a sialic group. The saccharide part is composed of at least one galactose or glucose and of at least one N-acetylglucosamine or N-acetylgalactosamine.

The general structure of a ganglioside can therefore be represented by the following formula:

one sialic acid mole { 
- one ceramide mole
- at least one galactose or glucose mole
- at least one mole of N-acetylglucosamine or N-acetylgalactosamine

in which these components are bound to glucoside bonds.

Numerous gangliosides have been identified which have proved to be particularly abundant in nerve tissues, especially in those of the brain.

Various studies have shown that the sialic groups most frequently encountered in gangliosides are N-acetylneuraminic acid (NANA) and, to a lesser degree, N-glycolylneuraminic acid. Among the many identified gangliosides, considerable amounts of the following gangliosides, classified by their international symbols, have been found in mixtures obtained from bovine brain tissue:

$GD_{1b}$ (16%)

Gal(1 $\xrightarrow{\beta}$ 3)GalNAC(1 $\xrightarrow{\beta}$ 4)Gal(1 $\xrightarrow{\beta}$ 4)Glc(1 $\xrightarrow{\beta}$ 1)Ceramide

(3 ↑ 2)

NANA

(8 ↑ 2)

NANA

$GT_{1b}$ (19%)

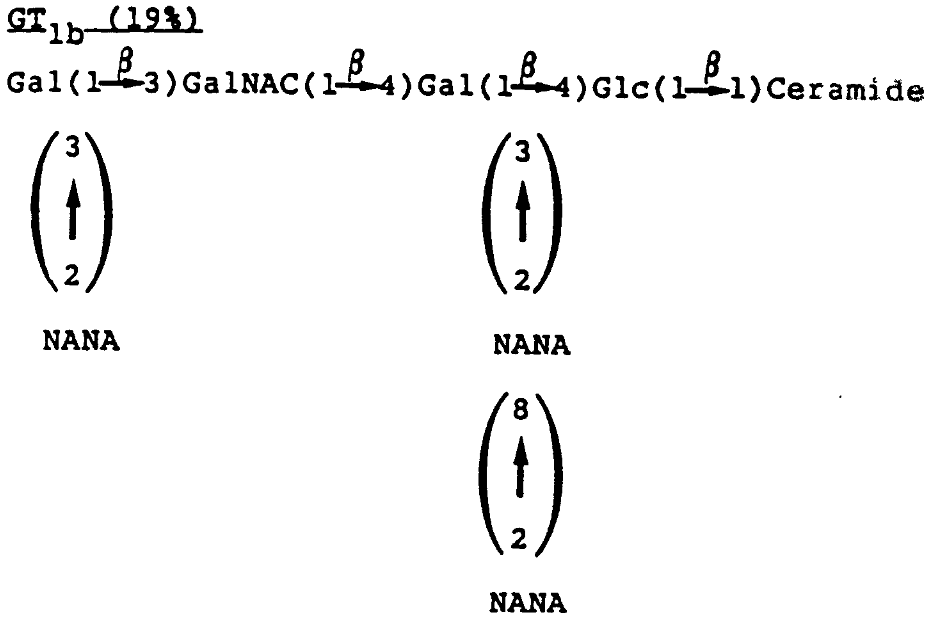

$GM_{1}$ (21%)

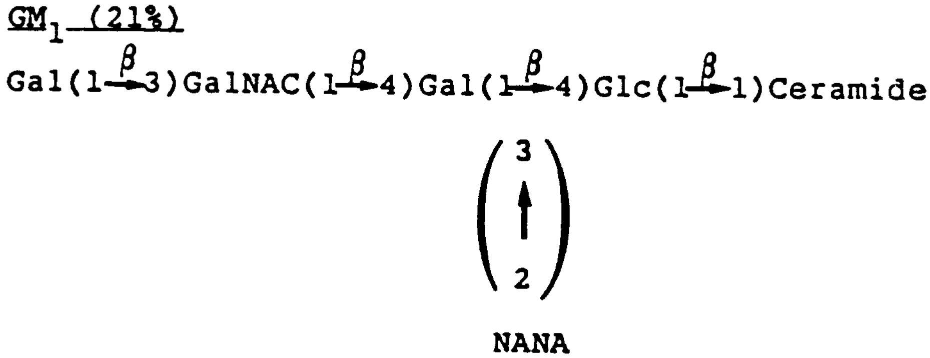

$GD_{1a}$ (40%)

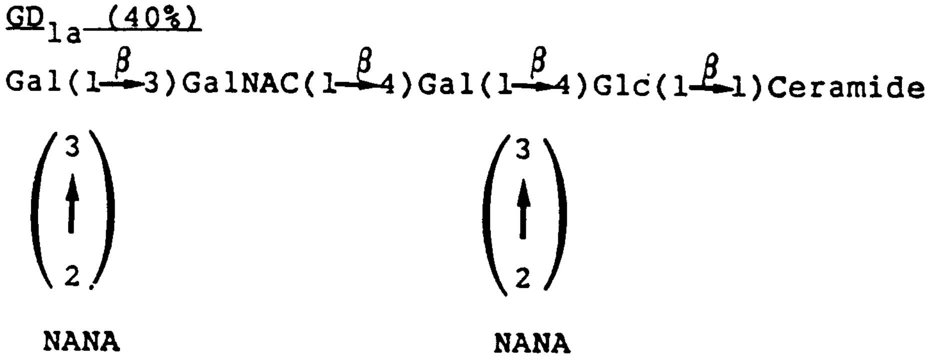

where Glc stands for glucose, GalNAc stands for N-acetylgalactosamine, Gal stands for galactose, NANA stands for N-acetylneuraminic acid and the percentages shown in brackets indicate the quantities relative to each ganglioside found in a ganglioside mixture extracted from bovine brain tissue.

The individual gangliosides reported above, isolated from a mixture extracted from animal tissues and considered to be quite "pure", still comprise mixtures due not only to the variability of their ceramide part, which corresponds to one of the formulae

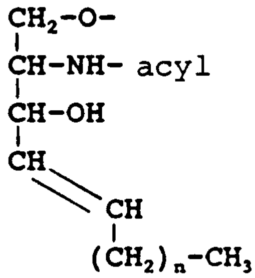 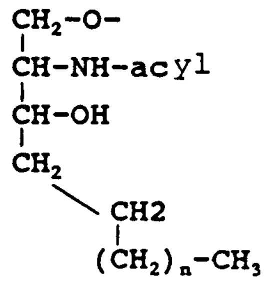

in which n = 10-16 and the acyl may derive from a saturated or unsaturated fatty acid having between 17 and 24 carbon atoms or from a corresponding hydroxy acid, but also to the variability of sialic acid, which may be either N-acetylneuraminic acid or N-glycolylneuraminic acid.

$GM_1$ ganglioside, whose isopropyl ester is the basis of the present invention, therefore represents also a mixture of compounds of the formula (I)

(I)

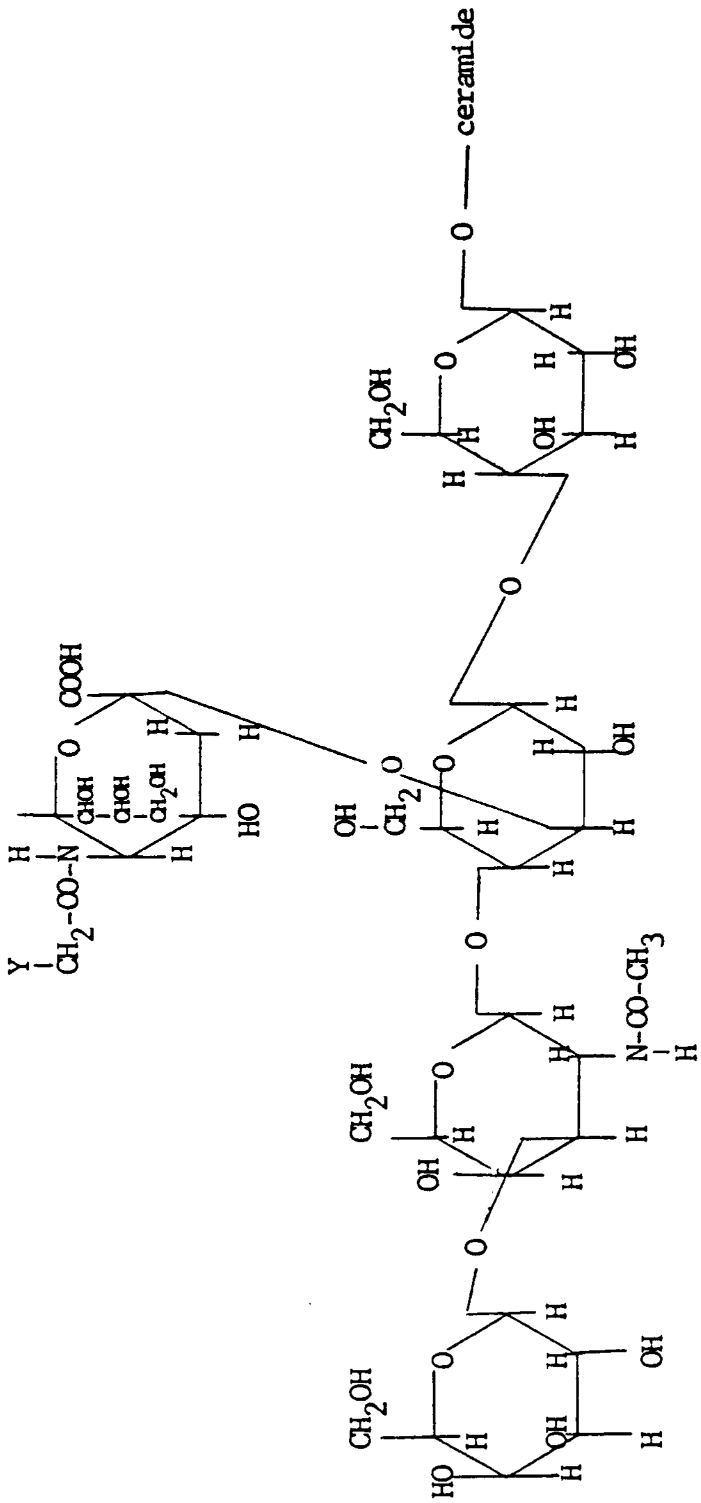

Y = H, OH

where "ceramide" represents a group of residues of the above formulae.

The isopropyl ester of $GM_1$ can, therefore be represented by the following formula (II):

(II)

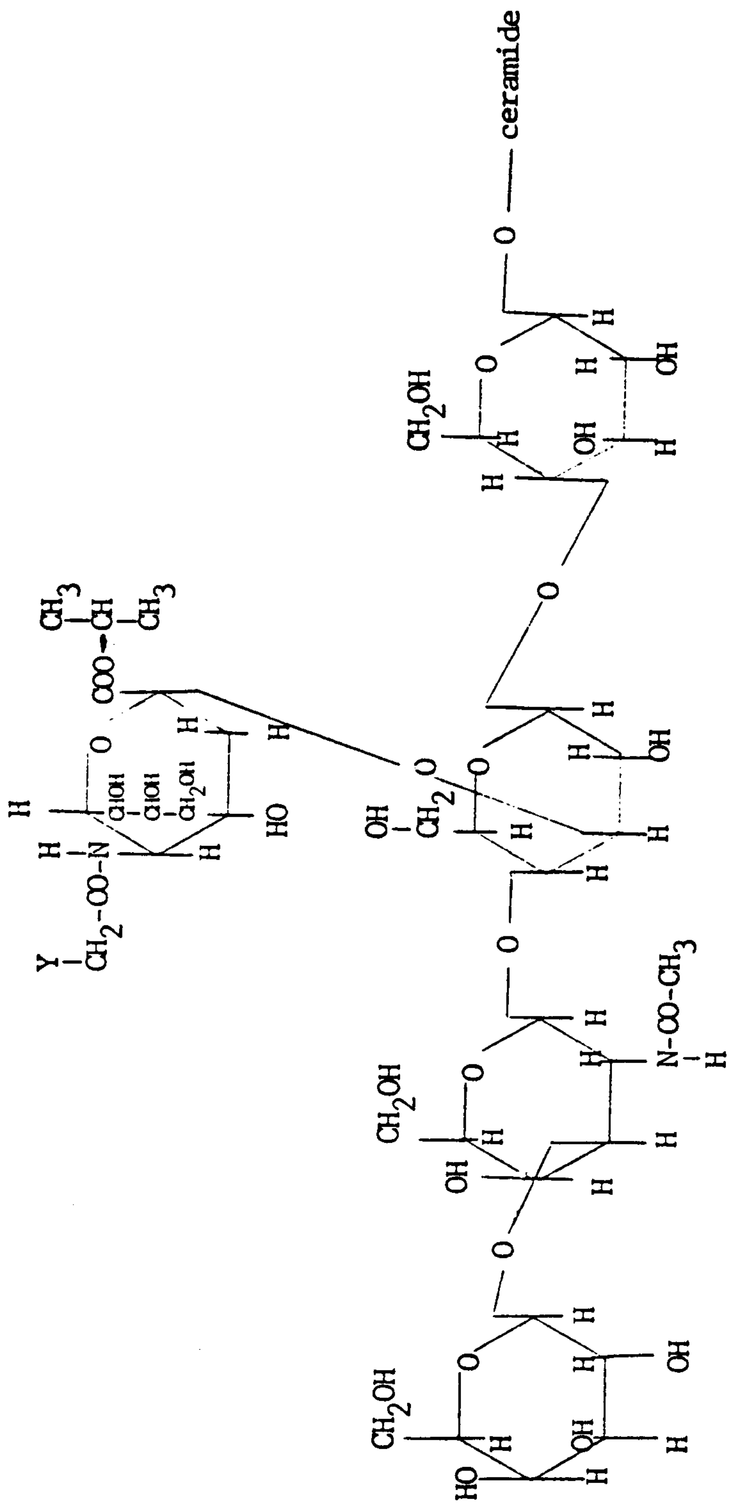

Y = H, OH

It is known that gangliosides play an important role in the nervous system and it has recently been demonstrated that gangliosides are useful in therapies for peripheral nervous system pathologies and in pathologies of the central nervous system.

The therapeutic action of gangliosides seems to consist above all in stimulating sprouting phenomena of the nerve cell and in activating the membrane enzymes involved in the conduction of nervous stimuli, such as the enzyme ($NA^+,K^+$)ATPase. Neuronal sprouting stimulated by gangliosides enhances functional

recovery of damaged nerve tissue, both central and peripheral.

It is also known that gangliosides, both in mixture and as single fractions, have a specific and high level of antinociceptive activity: they are effective in reducing writhings induced by phenylquinone and acetic acid and increased permeability induced by acetic acid, see EP 0 183 572.

In subsequent studies it has been demonstrated that a group of amides and esters of gangliosides have an advantage over gangliosides themselves in remaining active for a longer period of time ("retard" effect) and having more selective activity EP 0 167 449; Arch. Int. Pharmacodyn. 291, 238-252 (1988). It has now been observed that, apart from their antinociceptive action, ganglioside esters also present an antiinflammatory action which is quite dissociated from the first. Further studies described herein have brought to light valuable antiexudative and antiinflammatory properties of the isopropyl ester of $GM_1$ monosialoganglioside. The remarkable advantage of this ester is that while it can be administered orally, it is also active after topical administration.

As noted above, EP 0 167 449 describes methods for the preparation of ganglioside ester derivatives, including a procedure utilizing an internal ganglioside ester which is subsequently esterified with an alcohol. The $GM_1$ isopropyl ester is preferably prepared according to this procedure. However, other methods of obtaining the isopropyl ester may be based upon known esterification methods, such as alkylation reaction by means of isopropyl halogens in aprotic solvents or in mixtures of aprotic solvent, esterification by means of acidic catalysis and finally esterification catalyzed by ionic exchange resin.

The following, therefore, is an example of the preparation of the $GM_1$ isopropyl ester utilizing the internal ester process.

**Preparation Example 1: Preparation of isopropyl ester of GM1**

5 g of the internal ester of the ganglioside $GM_1$ (3.27 mM) are dissolved in 200 ml of an anhydrous mixture of methylene chloride and isopropyl alcohol 4:1. 176 mg (3.27 mM) of sodium isopropylate dissolved in 50 ml of anhydrous methanol are added and the mixture is refluxed for 2 hours. At the end of the reaction the mixture is neutralized with Dowex AG 50x8 anhydrous resin ($H^+$ form), the resin is separated by filtration and washed with isopropyl alcohol and the solution is evaporated by drying. The residue is gathered in 50 ml of methylene chloride/isopropanol 1:1 and the reaction product is precipitated by pouring it into 250 ml of acetone. The raw product (4.9g) is purified by preparative high pressure chromatography with 60 H Merck silica gel, using as solvent a mixture of chloroform/methanol/isopropanol/ammonium carbonate at 2% 1140:820:180:140. The pure fractions are gathered, evaporated by drying, redissolved in 15 ml of chloroform/methanol 1:1 and the product is precipitated with 75 ml of acetone. This product represents the isopropyl ester of the ganglioside $GM_1$. Yield 4.9g.

IR spectroscopy carried out on KBr pellets shows the typical bond of the ester at 1750 $cm^{-1}$. Chromatography on silica gel plates with chloroform/methanol/$CaCl_2$ at 0.3% 55:45:10 and determined with Ehrlich reagent shows the product to be a unitary compound with an Rf of 0.85 and free from the internal ester used as starting product (Rf 0.75) and from the ganglioside $GM_1$ (Rf 0.65). By treatment with an 0.1N solution of $Na_2CO_3$ for 60° for an hour, the ester bond is split, giving the primary product, $GM_1$.

The isopropyl ester of $GM_1$ ganglioside, as described in the above noted EP 0 167 449, is useful in therapies for peripheral nervous system pathologies of traumatic, compressive, degenerative or toxic infective origin, based upon the stimulation of nervous regeneration and neuromuscular functional recovery; and in pathologies of traumatic, anoxic,, degenerative or toxic infective origin in the central nervous system based upon stimulation of neuronal sprouting phenomena for functional recovery. It has now been found according to the present invention that the $GM_1$ isopropyl ester derivative may be used, due to its valuable anti-exudative and antiinflammatory properties, in systemic, ophthalmic and topical pathologies.

In the field of ophthalmology, the isopropyl ester derivative of $GM_1$ can be used in pathologies of diverse etiopathogenesis in which inflammatory phenomena are present and marked, such as: blepharitis, conjunctivitis, keratoconjunctivitis, keratitis, episcleritis, anterior, intermediate and posterior uveitis, sympathetic ophthalmia, retinitis, retinal vasculitis,, dysthyroid ophthalmopathy, and in post-surgical treatments following surgical operations to the bulbus oculi and annexa oculi.

For topical use, the isopropyl ester derivative of $GM_1$ is useful in pathologies of a nervous nature with an inflammatory component, such as lumbago, lumbo-sciatica, cervicobrachialgia in the various herpetic manifestations and infections such as herpes zoster.

This ganglioside derivative, like $GM_1$, has the important advantage of being administrable by oral route and of being remarkably efficacious by this route. Like $GM_1$, the ganglioside derivative can be used by topical route.

The isopropyl ester of $GM_1$ according to the present invention can be used as a drug in pharmaceutical preparations for administration to man or animal by oral, intramuscular, intravenous, subcutaneous or intradermal, topical, ophthalmic routes, by means of tablets, capsules, injections or intravenous infusions, gels, creams, ointments and eye drops. The preparations intended for oral administration can be prepared as powders or freeze-dried products mixed with one or more pharmaceutically acceptable excipients in the form of tablets or capsules. The preparations intended for administration as injections can be prepared as powders or freeze-dried products mixed with pharmaceutically acceptable excipients or diluents and contained in buffered solutions with a suitable pH and osmolarity compatible with the physiological fluids. The preparations intended for topical applications can be prepared as powders or freeze-dried products, mixed with one or more pharmacologically suitable excipients or vehicles and capable of permitting penetration of the active principle in the form of gels, creams, ointments. The preparations intended for ophthalmic administration can be prepared as powders or freeze-dried products mixed with one or more pharmaceutically acceptable excipients or diluents and contained in buffered solutions with suitable pH and osmolarity compatible with the ocular system.

The dosage to be administered depends on the desired effect and on the chosen administration route. As an example (not limitative) the dosage can be between 0.05 and 5 mg of active substance per kg of body weight/day with a unitary posology of between 0.05 and 2 mg/kg of body weight.

The following tables describe pharmaceutical compositions useful in the invention.

The types of pharmaceutical preparation shown in Table 1 are administrable by oral route and are represented by examples of tablets and capsules.

Example 1 describes a tablet obtained by direct compression of the components.

Example 3 describes a tablet obtained by damp granulation and subsequent compression.

Examples 2 and 4 report gastroresistant tablets.

Example 5 reports a capsule in hard gelatin.

**Table 1 - Examples of pharmaceutical preparations for oral administration**

Example 1

| | | | |
|---|---|---|---|
| - | active substance | mg | 25 |
| - | microcrystalline cellulose | mg | 100 |
| - | lactose | mg | 20 |
| - | maize starch | mg | 10 |
| - | talcum | mg | 5 |
| - | magnesium stearate | mg | 1.5 |

Example 2

Tablet described in Example 1 with a coating composed of:

| | | | |
|---|---|---|---|
| - | acetophthalate cellulose | mg | 4 |
| - | hydroxypropylmethylcellulose | mg | 0.2 |
| - | diethylphthalate | mg | 1.4 |
| - | shellac | mg | 1.5 |

Example 3

| | | | |
|---|---|---|---|
| - | active substance | mg | 50 |
| - | lactose | mg | 80 |
| - | maize starch | mg | 50 |
| - | talcum | mg | 3 |
| - | magnesium stearate | mg | 1.2 |

Example 4

Tablet described in Example 3 with a coating composed of:

| | | | |
|---|---|---|---|
| - | acetophthalate cellulose | mg | 4 |
| - | hydroxypropylmethylcellulose | mg | 0.2 |
| - | diethylphthalate | mg | 1.4 |
| - | shellac | mg | 1.5 |

Example 5

Granules comprising:

| | | | |
|---|---|---|---|
| - | active substance | mg | 50 |
| - | lactose | mg | 80 |
| - | maize starch | mg | 50 |
| - | talcum | mg | 3 |

| | | | |
|---|---|---|---|
| - | magnesium stearate | mg | 1.2 |

Hard gelatin involucre (Parke-Davis, etc.)

The type of pharmaceutical preparations shown in Table 2 are used for intramuscular, subcutaneous, intradermal or intravenous administration.

**Table 2 - Examples of pharmaceutical preparations:**

Example 1

| | | | |
|---|---|---|---|
| - | active substance | mg | 25 |
| - | sodium chloride | mg | 16 |
| - | phosphate buffer in distilled water q.b. | ml | 2 |

Example 2

| | | | |
|---|---|---|---|
| - | active substance | mg | 50 |
| - | mannitol | mg | 80 |
| - | phosphate buffer in distilled water q.b. | ml | 2 |

The type of pharmaceutical formulations shown in Table 3 relate to formulations to be used by topical route.

Example 1 describes an aqueous gel.

Example 2 describes a cream.

Example 3 describes an ointment.

**Table 3: Examples of pharmaceutical preparations (topical)**

Example 1

| | | |
|---|---|---|
| - | active principle | 1% |
| - | carbopol 940 | 1% |
| - | glycerol | 3% |
| - | methyl paraben | 0.2% |
| - | propyl paraben | 0.02% |
| - | purified $H_2O$ q.b. | 100 gr |

Example 2

| | | |
|---|---|---|
| - | active principle | 1% |

| | | |
|---|---|---|
| - | monostearate sorbitan | 1.5% |
| - | (2) OE monostearate sorbitan | 2.5% |
| - | vaseline oil | 5% |
| - | glycerol | 3% |
| - | methyl paraben | 0.2% |
| - | propyl paraben | 0.02% |
| - | purified $H_2O$ q.b. | 100 gr |

Example 3

| | | |
|---|---|---|
| - | active principle | 1% |
| - | white wax | 7% |
| - | spermaceti | 8% |
| - | sweet almond oil | 60% |
| - | purified $H_2O$ q.b. | 100 gr |

Table 4 reports examples of pharmaceutical formulations to be used by ophthalmic route in the form of eye drops.

**Table 4 - Examples of pharmaceutical preparations**

Example 1

| | | |
|---|---|---|
| - | active principle | 0.5% |
| - | NaCl | 0.85% |
| - | phosphate buffer in distilled water q.b. | 100 ml |

Example 2

| | | |
|---|---|---|
| - | active principle | 1% |
| - | NaCl | 0.85% |
| - | phosphate buffer in distilled water q.b. | 100 ml |

The above mentioned patent publication EP 0 167 449 describes ester derivatives of gangliosides, methods of producing such derivatives and their efficacy in therapies for nervous system pathologies. Ester derivatives of gangliosides may be used to treat a range of nervous disorders including pathologies of the peripheral and central nervous systems following disease or trauma. These substances may also be used in post-surgical therapies after operations involving motor and sensory nerves such as slipped disc operations.

One publication (Arch. Int. Pharmacodyn. 272, 103-117, (1984)) reports the antinociceptive properties of a ganglioside mixture which is able, after subcutaneous administration, to reduce the number of phenylquinone-induced writhings (and acetic acid-induced writhings) as well as increased permeability induced by acetic acid.

It has now been observed that the esterification of a ganglioside, particularly esterification of $GM_1$ with isopropyl alcohol, induces a dissociation of its antinociceptive and antiinflammatory properties. Indeed, the new derivative, isopropyl ester of $GM_1$, has a strong inhibiting effect on exudative and inflammatory reactions. It inhibits in a dose-dependent manner acetic acid-induced peritonitis, edema induced by carrageenan, serotonin, histamine and bradykinin in rat paw.

Its antiinflammatory activity is limited to a certain dose range only, suggesting an action mechanism of considerable interest.

In terms of the molar ratio, the isopropyl ester of $GM_1$ ganglioside is more active than the commercially available comparison drug.

Its antiedematous effect develops over a period of 30-60 minutes after induction of edema and reaches a peak within the first few hours, suggesting that mediators such as histamine, serotonin and bradykinin are involved in the process.

Apart from the systemic route (os, i.p., s.c.) the isopropyl ester derivative of $GM_1$ ganglioside presents antiexudative and antiinflammatory activity following topical application to the paw. After topical application to the paw, the new derivative proves to have an antiexudative activity; therefore, a certain systemic absorption is hypothesized.

In the case of $GM_1$ too, it is possible to observe antiexudative and antiinflammatory activities, although these activities do not reach the high percentages of inhibition reached by the isopropyl ester derivative.

The antiexudative and antiinflammatory activities comprising the object of the present invention can be evaluated by means of the following tests:

**ANTIEXUDATIVE ACTIVITY EVALUATED BY MEANS OF THE ACETIC ACID-INDUCED PERITONITIS TEST**

Procedure:

The study was conducted on Sprague-Dawley rats, weighing between 200 and 300 gr.

An evaluation was made of the capacity of the isopropyl ester of $GM_1$ ganglioside (hereafter known as $AGF_{44}$) and $GM_1$ itself, to inhibit the formation of peritoneal exudate following intraperitoneal injection (10 ml/kg) of a 0.5% acetic acid solution.

To study administration by oral, subcutaneous and intraperitoneal routes, $AGF_{44}$ and $GM_1$ (solubilized in 10 ml/kg of sterile saline physiological solution) and a comparison compound indomethacin were administered by the individual routes one hour before the injection of acetic acid.

To study topical administration, one hour before i.p. injection of acetic acid, $AGF_{44}$ and $GM_1$ in the form of 0.1% aqueous gels and the comparison 5% Artrosilene gel (ketoprofen lysine salt) were applied to the right paws with a light massage for 30 seconds to ensure complete absorption of the gel.

The control groups received saline physiological solution (10 ml/kg) and placebo gel (0.2 ml/rat), respectively. 30 minutes after exudate inducement the animals were sacrificed, samples were taken of the peritoneal exudate and the percentage of inhibition of the same in relation to control values was calculated.

Results:

The results are reported in Table 5. It is easy to see from the data presented, that $AGF_{44}$ has a remarkable dose-dependent antiexudative efficacy. The product proves to be particularly active at doses of between 1 and 5 mg/kg, but it is also efficacious at doses of less than 0.1 mg/kg (in the order of 0.1 mg/kg). At doses of over 10 mg/kg the activity decreases.

Peritoneal exudate inhibition is particularly strong after oral administration, when values of between 70-80% of inhibition are obtained. At 1 mg/kg the activity is comparable to that of indomethacin. After subcutaneous and intraperitoneal treatment, the anti-exudative effect is evident, even though the inhibition values reached are slightly inferior (50-60%). Further interest in this compound derives from analysis of the results of topical treatment where an antiexudative activity is present also when $AGF_{44}$ is applied to the paw in the form of an aqueous gel. It is hypothesized that these results suggest a certain systemic absorption is hypothesized. In the same way, $GM_1$ presents a marked antiexudative activity by all the administration routes tested, above all in the case of oral administration, without however, reaching the high percentages of inhibition reached by the isopropyl ester.

**Table 5:**

Results of the acetic acid-induced peritonitis test in rat, using an i.p. injection (10 ml/kg) of a solution of 0.5% acetic acid.

| Treatment | dose (mg/kg) | No. animals | % inhibition |
|---|---|---|---|
| Oral | | | |
| Controls (saline phys. solution 10 ml/kg) | - | 60 | - |
| $GM_1$ isopropyl ester | 1 | 12 | 70 |
| $GM_1$ isopropyl ester | 2.5 | 12 | 72 |
| $GM_1$ isopropyl ester | 5 | 12 | 77 |
| $GM_1$ | 1 | 12 | 56 |
| $GM_1$ | 2.5 | 12 | 63 |
| Indomethacin | 1 | 12 | 74 |
| Indomethacin | 2.5 | 12 | 90 |
| Subcutaneous | | | |
| Controls (saline phys. solution 10 ml/kg) | - | 60 | - |
| $GM_1$ isopropyl ester | 1 | 12 | 47 |
| $GM_1$ isopropyl ester | 2.5 | 12 | 61 |
| $GM_1$ isopropyl ester | 5 | 12 | 70 |
| $GM_1$ | 1 | 12 | 44 |
| $GM_1$ | 2.5 | 12 | 52 |
| Indomethacin | 1 | 12 | 64 |
| Indomethacin | 2.5 | 12 | 84 |

**Table 5** (cont'd)

| Treatment | dose (mg/kg) | No. animals | % inhibition |
|---|---|---|---|
| *Intraperitoneal* | | | |
| Controls (saline phys. solution 10 ml/kg) | - | 60 | - |
| $GM_1$ isopropyl ester | 1 | 12 | 55 |
| $GM_1$ isopropyl ester | 2.5 | 12 | 60 |
| $GM_1$ isopropyl ester | 5 | 12 | 64 |
| $GM_1$ | 1 | 12 | 47 |
| $GM_1$ | 2.5 | 12 | 51 |
| Indomethacin | 1 | 12 | 70 |
| *Topical* | | | |
| Controls (placebo gel 0.2 ml/rat) | - | 40 | - |
| $GM_1$ isopropyl ester 0.1% gel (0.2 ml/rat) | 1.4 | 20 | 43 |
| $GM_1$ 0.1% gel (0.2 ml/rat) | 1.4 | 20 | 30 |
| Artrosilene 5% gel 0.4 ml/rat) | 70* | 20 | 40 |

* dose expressed as ketoprofen acid

**ANTIEDEMATOUS ACTIVITY EVALUATED BY THE CARRAGEENAN-INDUCED EDEMA TEST IN THE PAW**

Procedure:

The study was conducted on male Sprague Dawley rats, weighing about 160 ± 10 g.

An evaluation was made of the activity of the isopropyl ester of $GM_1$ ganglioside ($AGF_{44}$) and $GM_1$ in inhibiting the formation of edema following injection of a sterile aqueous solution of 1% carrageenan into the

plantar aponeurosis of the right hand paw (0.1 ml/rat).

To study administration by oral, subcutaneous and intraparitoneal routes, $AGF_{44}$ and $GM_1$ (solubilized in 10 ml/kg of sterile saline physiological solution) were compared to indomethacin as a comparison compound. For study of the oral route, each compound was administered by oral route one hour before injection of carrageenan. For study of the subcutaneous and intraperitoneal routes, each compound was administered 30 minutes before the same. To study topical administration, $AGF_{44}$ and $GM_1$ (as 0.1% aqueous gels) were compared to 5% Artrosilene gel (ketoprefene lysine salt). Each compound was applied locally to the right paw, and administration was effected twice, one hour before and immediately after injection of carrageenan, massaging lightly for 30 seconds to ensure complete absorption.

The control groups received saline physiological solution (10 ml/kg) and placebo gel (0.2-0.4 ml/rat twice) respectively.

Plethysmometric evaluation of the volume of the right paw was effected immediately before sub-plantar injection of carrageenan an 3 hours after the same. The antiedematous effect of the treatment was calculated by evaluating the average increase in paw volume at the 3rd hour (compared to time 0) based on control values.

Results:

The results are reported in Table 6.

**Table 6:**

**Results of the carrageenan-induced edema test by means of injection into the plantar aponeurosis of the right hind paw of a sterile aqueous solution of 1% carrageenan (0.1 ml/rat).**

| Treatment | dose (mg/kg) | No. animals | % inhibition |
|---|---|---|---|
| **Oral** | | | |
| Controls (saline phys. solution 10 ml/kg) | - | 60 | - |
| $GM_1$ isopropyl ester | 1 | 12 | 20 |
| $GM_1$ isopropyl ester | 2 | 12 | 32 |
| $GM_1$ isopropyl ester | 5 | 12 | 60 |
| $GM_1$ | 2 | 12 | 20 |
| $GM_1$ | 5 | 12 | 32 |
| Indomethacin | 1 | 12 | 22 |
| Indomethacin | 5 | 12 | 67 |
| **Intraperitoneal** | | | |
| Controls (saline phys. solution 10 ml/kg) | - | 60 | - |
| $GM_1$ isopropyl ester | 1 | 12 | 18 |
| $GM_1$ isopropyl ester | 2 | 12 | 32 |
| $GM_1$ isopropyl ester | 5 | 12 | 57 |
| $GM_1$ | 2 | 12 | 20 |
| $GM_1$ | 5 | 12 | 30 |
| Indomethacin | 1 | 12 | 23 |
| Indomethacin | 5 | 12 | 67 |

Table 6 (cont'd)

| Treatment | dose (mg/kg) | No. animals | % inhibition |
|---|---|---|---|
| **Subcutaneous** | | | |
| Controls (saline phys. solution 10 ml/kg) | - | 60 | - |
| $GM_1$ isopropyl ester | 1 | 12 | 25 |
| $GM_1$ isopropyl ester | 2 | 12 | 30 |
| $GM_1$ isopropyl ester | 5 | 12 | 50 |
| $GM_1$ | 2 | 12 | 19 |
| $GM_1$ | 5 | 12 | 31 |
| Indomethacin | 1 | 12 | 30 |
| Indomethacin | 5 | 12 | 60 |
| **Topical** | | | |
| Controls (placebo gel 0.2 ml/rat) | - | 40 | - |
| Controls (placebo gel 0.4 ml/rat) | - | 40 | - |
| $GM_1$ isopropyl ester 0.1% gel (0.2 ml/rat/twice) | 1.25 x 2 | 12 | 32 |
| $GM_1$ isopropyl ester 0.1% gel (0.4 ml/rat/twice) | 2.5 x 2 | 12 | 43 |
| $GM_1$ 0.1% gel (0.2 ml/rat/twice) | | 12 | 20 |
| Artrosilene 5% gel 0.2 ml/rat/twice) | 70* | 12 | 38 |

* dose expressed as ketoprofen acid

The efficacy of $AGF_{44}$ in inhibiting carrageenan-induced edema in the paw is evident, although in this test it does not reach such high percentages of inhibition as in the acetic acid-induced peritonitis test. Its antiinflammatory activity is effective at low doses (it diminishes at doses of over 10 mg/kg), and develops within the first few hours after edema inducement, suggesting that the antiinflammatory activity is aimed at effects related to histamine,, serotonin and bradykinin release following damage.

In the carrageenan-induced edema test, $AGF_{44}$ is active by all administration routes tested: oral, subcutaneous, intraperitoneal, topical. Maximum efficacy is reached by oral route (50-60% inhibition). Of great interest were the results obtained from topical treatment, because in that test $AGF_{44}$ applied to the paw has an antiedematous activity even at the lowest dose tested.

In the case of $GM_1$ too, an antiedematous activity is present by the the various administration routes tested. The efficacy, however, is inferior to that observed for the ester derivative.

**Claims**

1. Use of the isopropyl ester of $GM_1$ ganglioside for the manufacture of a medicament for the treatment of the inflammatory and exudative components of nerve pathologies.

2. Use of the isopropyl ester of $GM_1$ ganglioside for the manufacture of a medicament for the treatment of inflammation or exudation caused by systemic, ophthalmic or topical pathologies.

3. Use of the isopropyl ester of $GM_1$ ganglioside according to one of claims 1 or 2 for the manufacture of an ophthalmic medicament for the treatment of the inflammatory phenomena of ophthalmological pathologies.

4. Use of the isopropyl ester of $GM_1$ ganglioside according to claim 3 for the manufacture of a medicament for the treatment of inflammation or exudation caused by blepharitis, conjunctivitis, keratoconjunctivitis, keratitis, episcleritis, anterior, intermediate and posterior uveitis, sympathetic ophthalmia, retinitis, retinal vasculitis, dysthyroid ophthalmopathy and for postoperative treatments following surgical operations to the bulb and appendages.

5. Use of the isopropyl ester of $GM_1$ ganglioside according to one of claims 1 or 2 for the manufacture of a topical medicament for the treatment of the inflammatory component of nerve pathologies.

6. Use of the isopropyl ester of $GM_1$ ganglioside according to claim 5 for the manufacture of a medicament for the treatment of inflammation or exudation caused by lumbago, lubosciatica, cervicobrachialgia and herpes zoster.

7. Use of the isopropyl ester of $GM_1$ ganglioside according to one of claims 1-3 for the manufacture of a medicament for oral administration.

8. Use of the isopropyl ester of $GM_1$ ganglioside according to one of claims 4 or 6 for the manufacture of a medicament for topical administration.

**Patentansprüche**

1. Verwendung des Isopropylesters von $GM_1$-Gangliosid zur Herstellung eines Medikaments für die Behandlung von entzündlichen und exsudativen Aspekten von Nervenerkrankungen.

2. Verwendung des Isopropylesters von $GM_1$-Gangliosid zur Herstellung eines Medikaments für die Behandlung einer Entzündung oder Exsudation, die durch systemische, ophthalmische oder topische Erkrankungen verursacht ist.

3. Verwendung des Isopropylesters von $GM_1$-Gangliosid nach einem der Ansprüche 1 oder 2 zur Herstellung eines ophthalmischen Medikaments für die Behandlung von entzündlichen Phänomenen bei ophthalmologischen Erkrankungen.

4. Verwendung des Isopropylesters von $GM_1$-Gangliosid nach Anspruch 3 zur Herstellung eines Medikaments für die Behandlung einer Entzündung oder Exsudation, die durch Blepharitis, Conjunctivitis, Keratoconjunctivitis, Keratitis, Episcleritis, Uveitis anterior, intermediär und posterior, sympathische Ophthalmie, Retinitis, retinale Vasculitis, mit Funktionsstörungen der Schilddrüse einhergehende Ophthalmopathien und für die postoperativen Behandlungen nach Operationen am Augapfel und den Appendices.

5. Verwendung des Isopropylesters von $GM_1$-Gangliosid nach einem der Ansprüche 1 oder 2 zur Herstellung eines topischen Medikaments für die Behandlung des Entzündungsaspekts von Nervenerkrankungen.

6. Verwendung des Isopropylesters von $GM_1$-Gangliosid nach Anspruch 5 für die Herstellung eines Medikaments zur Behandlung einer Entzündung oder Exsudation, die durch Lumbago, Lumbalischias, Cervicobrachialgie und Herpes zoster verursacht wird.

7. Verwendung des Isopropylesters von $GM_1$-Gangliosid nach einem der Ansprüche 1 bis 3 zur Herstellung eines Medikaments für die orale Verabreichung.

8. Verwendung des Isopropylesters von $GM_1$-Gangliosid nach einem der Ansprüche 4 oder 6 zur Herstellung eines Medikaments für die topische Verabreichung.

## Revendications

1. Utilisation de l'ester isopropylique de ganglioside $GM_1$ pour le fabrication d'un médicament destiné au traitement des composantes inflammatoires et exudatives de pathologies neurologiques.

2. Utilisation de l'ester isopropylique de ganglioside $GM_1$ pour la fabrication d'un médicament destiné au traitement d'inflammation ou d'exudation provoquées par des pathologies systémiques, ophtalmiques ou topiques.

3. Utilisation de l'ester isopropylique de ganglioside $GM_1$ selon l'une des revendications 1 ou 2 pour la fabrication d'un médicament ophtalmique destiné au traitement de phonomènes inflammatoires de pathologies ophtalmologiques.

4. Utilisation d'ester isopropylique de ganglioside $GM_1$ salon la revendication 3, pour la fabrication d'un médicament destiné au traitement d'inflammation ou d'exudation provoquées par la blépharite, la conjonctivite, la kératoconjonctivite, la kératite, l'épisclérite, l'uvéite antérieure, intermédiaire et postérieure, l'ophtalmie symphatique, la rétinite, la vasculite rétiniène, l'ophtalmophatie dysthyroïdienne et destiné aux traitements postopératoires suivant les interventions chirurgicales sur le bulbe et les appendices.

5. Utilisation de l'ester isopropylique de ganglioside $GM_1$ selon l'une des revendications 1 ou 2 pour la fabrication d'un médicament topique destiné au traitement de la composante inflammatoire de pathologies neurologiques.

6. Utilisation d'ester isopropylique de ganglioside $GM_1$ selon la revendication 5, pour la fabrication d'un médicament destiné au traitement d'inflammation ou d'exudation provoquées par un lumbago, une lumbosciatique, une cervicobrachialgie et un herpès zoster.

7. Utilisation d'ester isopropylique de ganglioside $GM_1$ selon l'une des revendications 1-3 pour la fabrication d'un médicament destiné à l'administration orale.

8. Utilisation de l'ester isopropylique de ganglioside $GM_1$ selon l'une des revendications 4 ou 6 pour la fabrication d'un médicament destiné à l'administration topique.